# EUROPEAN PATENT SPECIFICATION

(11) **EP 0 574 038 B1**
(45) Date of publication and mention of the grant of the patent: **02.02.2000**
(21) Application number: 93113073.6
(22) Date of filing: 26.09.1989
(51) Int. Cl.: A61M 15/00

(54) **Medication delivery apparatus**
Gerät zum Verabreichen von Medikamenten
Dispositif pour la délivrance de médicaments

(30) Priority: 30.09.1988 US 251454
(43) Date of publication of application: 15.12.1993
(62) Divisional of application: 89309757.6
(73) Proprietor: THE JOHNS HOPKINS UNIVERSITY, Baltimore, MD 21218 (US)
(72) Inventor: Zoltan, Bart Joseph, Old Tappan, New Jersey 07675 (US); Laube, Beth Louise, Baltimore, Maryland 21239 (US); Adams III, George Kenneth, Baltimore, Maryland 21212 (US)
(74) Representative: Dean, John Paul

(56) References cited:
- US-A- 2 788 784
- US-A- 4 534 343

## Description

### BACKGROUND OF THE INVENTION

### Field of the Invention

This invention relates to the dispensing of pharmaceutical which are active when administered as aerosols and, more specifically, to the dispensing of aerosol pharmaceutical for the treatment of pulmonary diseases.

Certain medications, especially those intended for the treatment of acute and chronic respiratory disorders, for example, bronchodilator which are used in the treatment of bronchospasm and mucosal edema, are most effective when inhaled directly into the lungs. Similarly, antibiotic aerosols used to treat bronchial infections, antiinflammatory steroids used for the treatment of asthma, antifoaming agents for the treatment of fulminant pulmonary edema, and cromolyn sodium for controlling allergic asthma are most effective when administered in this manner. Thus, numerous pharmaceutical products are marketed as aerosols administered from metered dose inhalers.

While aerosol therapies have been very successful, there have been a number of difficulties in dispensing the aerosols properly.

A major problem of aerosol therapy is achieving deposition of the aerosol on the walls of small bronchi and brochioles, where the action of the medication is most often required. Less than the percent of the medication delivered by standard metered dose inhalers reaches the typical patient's lungs. Most of the ninety percent of the medication which does not penetrate to the target area is deposited in the mouth, throat, and trachea, and is eventually ingested, A small fraction of the aerosol is exhaled. The medication which deposits in the mouth and throat may lead to dysphonia and/or oral and laryngeal candidiasis while the medication which is ingested serves no medical purpose to the patent and is responsible only for undesirable side effects.

Therefore the delivery of some drugs via aerosol has been considered impractical. Nevertheless the aerosol delivery of many drugs, if possible, would present an attractive alternative to the therapies that are currently available. An example of such a substance is polypeptides.

Polypeptides are made up of amino acid sequences, and include large molecules like insulin, and all of the products of recombinant DNA (rDNA) techniques. These molecules are broken down in the digestive tract and, therefore, the intact polypeptide molecule is not absorbed into the bloodstream. Accordingly, the only practical way to administer these drugs is by injection, although the nasal route of administration would be desirable and has been suggested, but has not been practical.

Another example is tissue plasminogen activator (t-PA) which appears to be successful in halting damage done to cardiac muscle during myocardial infarction. There could be an advantage to being able to utilize this drug as an aerosol for inhalation so that it could be administered without the need to wait for a physician or paramedic.

Delivery of therapy in pneumonia directly to the lung would also be desirable. Ordinarily, the concentration of antibiotic in the sputum is only two to three percent of the concentration in blood. However, in pneumonia, antibiotic concentration in the sputum is believed to be the determining factor for efficacy of the therapy. Therefore, direct delivery of the antibiotic may improve the effectiveness of the treatment.

In order to avoid the problems encountered with aerosol delivery, noted above, the aerosol should consist of small particles, less than 5 microns, since larger particles cannot negotiate the sharp turns to the lung and are deposited in the oropharynx due to inertial effects. Particles that persist in the airstream beyond the oropharynx may deposit in the larynx and on the walls of the trachea and large bronchi as a result of turbulence, particularly if the patient inhales at a volumetric flow rate above 30 litres per minute.

Metered dose inhalers are known, for example, in US-A-4,534,343, deliver aerosol at a very high velocity directly into the patient's mouth, and most of the medication impacts and is deposited in the mouth. This high initial velocity of the aerosol is a major factor in the ineffectiveness of many inhaler systems. In order to minimize mouth deposition it has been determined that the volumetric flow rate of the inhaled aerosol should be below 30 litres per minute.

After the medication has been inhaled it is best to continue inhaling to total lung capacity to promote greater penetration of drug to the lung periphery. One should then hold that breath for four to ten seconds, if possible, to allow for sedimentation of particles onto the airway surface.

Several pharmaceutical manufacturers have included, or sold separately with their aerosol products, what they refer to variously as "spacers", "inhalers", "drug inhalers", "oral adapters", "spacer-inhalers", and "spray inhalers" to be used in conjunction with their products.

Of the related devices known to the inventors, only Sackner et al., U.S. Patent No. 4,484,577, marketed as the InspirEase (from Key Pharmaceutical, Inc., Miami, Florida) addresses the known problems of aerosol inhalation. The InspirEase is essentially a collapsible bag into which the medication is metered, and from which the patient inhales. The InspirEase mouthpiece contains a whistle which is silent at low flow rates but sounds when the patient is inhaling too rapidly.

Further, laboratory equipment has been developed which allows inspired air to be measured using a pneumotachograph. The flow rate signal is integrated by a computer, and an aerosol canister containing the medication is actuated automatically at a predetermined lung volume using a solenoid mounted on top of the aerosol actuator. While this system is suitable for experimental studies, it is impractical for use in routine therapy because of size and cost.

Thus, there is a need for a device to aid patients in taking their aerosol medications. The device should limit the volumetric flow rate of the medication aerosol as it enters the mouth, and it should allow the patient to inhale to total lung capacity. The size of the device should allow it to be carried by the patient without too much inconvenience, and the cost to the patient should be low.

### SUMMARY OF THE INVENTION

Briefly, the apparatus described in United States Patent No. 4, 790,305 delivered a volume of unmedicated air from a collapsible holding chamber to the patient, after which it automatically began to deliver the aerosolized medication. The volumetric flow rate of the inhaled medication was maintained below the upper limits of volumetric flow rate for optimal dosing. While that design met all of the criteria for a safe and effective design, the method of holding a volume of unmedicated air can be very cumbersome.

In addition, there is lack of agreement as to the precise optimum in lung volume at the time of inhalation that will maximize the benefit from inhaled aerosols. In the medical literature, the optimal lung volume that is recommended ranges from 20 percent to 80 percent of vital capacity.

The apparatus described in this application eliminates the cumbersome volume of unmedicated air altogether, while it overcomes some of the disadvantages associated with prior attempts, and provides the patient and his physician with a practical means of taking aerosol medication in an optimal manner at low cost. This is achieved by metering the aerosol medication into a rigid chamber, from which the patient inhales the medication. Accordingly, the present invention provides an apparatus for delivering medication to a patient, comprising: a mouthpiece the proximal end of which is shaped to be accommodated by a human mouth; and a rigid housing having a first end a second end and a main body portion defining a chamber therewithin, said first end having an aperture defined therethrough and in communication with an interior of said mouthpiece so that said chamber is fluidly coupled to said mouthpiece through said aperture in said first end, the rigid housing has a second aperture through which medication can be introduced into the chamber of the housing, characterised in that said rigid housing further has at least one orifice defined through at least one of said first end said second end and said main body portion for limiting the volumetric flow rate of air into said chamber whereby once medication has been delivered to the interior of the rigid housing a patient breathing through the mouthpiece can inhale medicated air from the chamber and then continue inhaling to his total lung capacity at a limited volumetric flow rate defined by said at least one orifice. The chamber is of a flow-through design, so that the patient may continue to inhale from the chamber even after the initial volume has been inspired. Throughout this specification, the term rigid chamber refers to the chamber while in use. Indeed, for the sake of portability, it is desirable to fold or collapse the subject invention into a small space. Thus the term rigid is meant to be applied in the same way that an umbrella may be described as rigid when in use, although it can be stored in a closed configuration and extended prior to use.

The volumetric flow rate is limited by the use of orifices at the back of the chamber. The orifices are nominally sized for the typical frail patient, with provisions for closing one or several orifices if the physician or the patient feels that the nominal setting is inappropriate for a specific patient.

In use, the aerosol medication is metered into the rigid chamber. The patient inhales through a single mouthpiece. As the patient begins to inhale from the rigid chamber containing the medication, his volumetric flow rate is limited to 30 liters per minute by the orifices described above. The patient continues to inhale until he reaches his total lung capacity, at which time he holds his breath for the time recommended by his physician.

Other objects, features and characteristics of the present invention, as well as the methods of operation and functions of the related elements of the structure, and the combination of parts and economies of manufacture, will become more apparent upon consideration of the following detailed description and the appended claims with reference to the accompanying drawings all of which form a part of this specification, wherein like reference numerals designate corresponding parts in the various figures.

### BRIEF DESCRIPTION OF THE DRAWINGS

Fig. 1A is a perspective view of the mouthpiece of the invention;
Fig. 1B is a cross-sectional view of the mouthpiece of the present invention;
Fig. 2A is a cross-sectional view of the drawing of an embodiment of the invention, wherein the source of the aerosol, and the mouthpiece are not integral to the invention;
Fig. 2B is an elevational view of the end of the chamber of Fig. 2A;
Fig. 3A is an elevational view of an alternate embodiment of the invention, wherein the device is collapsible;
Fig. 3B shows the device of Fig. 3A in its collapsed configuration;

### DETAILED DESCRIPTION OF THE PRESENTLY PREFERRED EXEMPLARY EMBODIMENTS

The mouthpiece 10 of the invention is shown in FIG.s 1A and 1B. The proximal end 12 of the mouthpiece 10 is shaped to be accommodated in the mouth of the patient. In the preferred embodiment, a standard aerosol medication is administered from a metered dose inhaler 14 which is mounted to a coupling 16, which is designed to accommodate metered dose inhalers. Passage 18 directs the aerosol from the metered dose inhaler 14 out through the distal end of the mouthpiece. The distal end of the mouthpiece also has keys 20a and 20b, for example, which allow the mouthpiece 10 to be attached to the rigid chamber 30.

The invention was evaluated in a normal subject at the Johns Hopkins University Hospital. The results of this evaluation are reported below.

The normal subject was studied at the same time of day on two study days. On the first study day, the subject inhaled a 0.9% saline solution containing the radioisotope Tc-99m sulfur colloid ad libitum using prior art device, a DeVilbiss #42 nebulizer.

This nebulizer was connected to a 1.37 bar (20 p.s.i.) compressed air source through a nebulization dosimeter (Laboratory for Applied Immunology - Baltimore, Maryland), which controlled the duration of compressed air pulse during aerosol generation. A manual trigger, controlled by the subject, was used to actuate the nebulizer air flow for a period of 0.6 seconds.

On the second study day, the subject inhaled the same radioaerosol using the #42 DeVilbiss nebulizer in conjunction with the present invention. Actuation of the nebulizer airflow was manually triggered, at which time radioaerosol was delivered to the rigid chamber of the subject invention. The subject inhaled the radioaerosol from the rigid chamber to total lung capacity, at which time the subject exhaled slowly with no breath holding.

Following the inhalation of the radioaerosol, the subject's left lateral skull and lungs were scanned for ten minutes each with a Technicare Model #110 gamma camera.

Counts from the gamma camera were acquired in a 26 by 256 picture element (pixel) matrix and were processed for oropharyngeal and bronchopulmonary deposition in a 64 by 64 pixel matrix using an Informatek SIMIS 2 computer.

Aerosol deposited in the oropharynx and the bronchopulmonary region was expressed as a percent of total counts in the two regions.

Intrapulmonary aerosol deposition was further quantified in terms of aerosol initially deposited within an inner, intermediate, and outer zones of the right lung (anterior image). These zones are circular crescents dividing the radius of the right lung to its outer boundary into three equal segments. The left anterior lung image was not used for this analysis because its inner and intermediate zone activities are attenuated by the heart. Counts within each of the three lung zones were divided by the number of picture elements (pixels) in each zone, mean counts/pixel, and the mean counts/pixel in each zone were compared. For this analysis it was assumed that the inner zone is comprised predominantly of large central airways; while the outer zone reflects terminal airways and alveoli.

The intermediate zone includes a combination of large and small airways as well as alveolar regions of the lung. An inner-to-outer ratio of counts per pixel of 1.00 would indicate a homogenous distribution of the aerosol within the lungs. The scan images were photographed directly from the computer display using a 35mm camera. Scan photos were reproduced for display as glossy prints on high contrast film.

Table 1 shows the regional analysis of the anterior lung images of radiolabelled aerosol deposition using the DeVilbiss #42 nebulizer, and the DeVilbiss #42 nebulizer together with the subject invention. A reduction in the Inner:
Outer zone ratio following administration of the radioaerosol using the DeVilbiss #42 nebulizer with the subject invention indicates an increase in distribution homogeneity within the lungs.

**TABLE 1**

| REGIONAL DEPOSITION OF RADIOLABELLED AEROSOL | | |
|---|---|---|
| | DeVilbiss #42 nebulizer | DeVilbiss #42 nebulizer with the subject invention |
| Right inner lung: | | |
| Counts | 3,158 | 2,570 |
| No. of pixels | 75 | 75 |
| Counts per pixel | 42.11 | 34.27 |

| Right middle lung: | | |
|---|---|---|
| Counts | 4,682 | 7,254 |
| No. of pixels | 201 | 201 |
| Counts per pixel | 23.29 | 36.09 |

| Right outer lung: | | |
|---|---|---|
| Counts | 2,284 | 6,274 |
| No. of pixels | 247 | 247 |
| Counts per pixel | 9.25 | 25.29 |

| Ratio of counts per pixel: | | |
|---|---|---|
| Inner:/Outer | 4.35 | 1.36 |
| Inner:/Middle | 1.81 | 0.95 |
| Middle:/Outer | 2.52 | 1.43 |

Table 2 shows the statistical distribution of the deposition of the radiolabelled aerosol using the Devilbiss #42 nebulizer, and the DeVilbiss #42 nebulizer together with the subject invention. A reduction in values of skew (a measure of distribution asymmetry) and Kurtosis (a measure of distribution range) following administration of the radioaerosol with the DeVilbiss #42 nebulizer in conjunction with the subject invention indicates an Increase in aerosol distribution homogeneity within the lungs.

**TABLE 2**

| STATISTICS OF THE RADIOLABELLED IMAGES | | |
|---|---|---|
| | DeVilbiss #42 nebulizer | DeVilbiss #42 Nebulizer with the subject invention |
| Mean counts per picture element | 10.60 | 21.18 |
| Standard deviation | 8.79 | 12.88 |
| Skew | 2.64 | 0.43 |
| Kurtosis | 10.21 | 1.74 |

FIG.s 2A and 2B show an embodiment of the invention. A rigid chamber 50, is provided having a proximal end 52, and a distal 54, and a main body 58 therebetween. The proximal end 52 has an aperture 56, which is sized to accept standard disposable mouthpieces currently in use with hospital nebulizers. The body of the device 58 also has an opening 62 of a size suitable for coupling to an external source of aerosolized medication thereto.

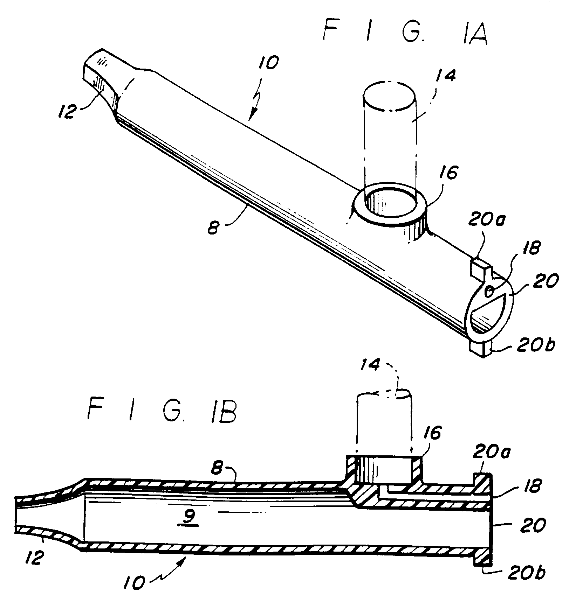

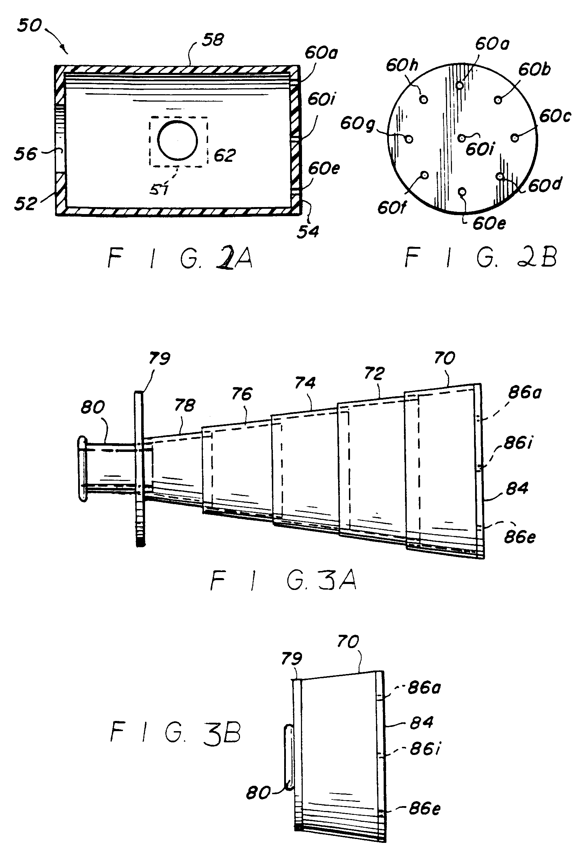

A means for closing the opening 62 is further provided in accordance with the present invention. If the external source of aerosolzed medication is retained in the opening 62 then that container will close the opening 62. In the alternative, the opening 62 can be closed after delivery of medicament to the chamber. The means for closing is shown schematically by phantom box 57 and may be any conventional means for closing such as, for example, a piece of tape or a one way valve structure. The distal end of the device 54, has a multiplicity of flow restricting orifices 60a-i (FIG. 2B).

Another alternate embodiment of the invention is shown in FIG.s 3A and 3B. The rigid chamber in this embodiment is collapsible. thereby making the invention more portable, and less noticeable when carried. Many patients especially paediatric patients are embarrassed by their need to take medication, and this embodiment minimizes size and obtrusiveness. In FIG. 3A, the device is shown in its extended form, reads for use. The largest component piece, a truncated cone segment 70, has rate limiting orifices shown as 86a-c. Other similar offices are provided but not shown in particular. Truncated cone segments 70-78, nest inside one another and are thereby collapsible.

Segment 78 also has a circular flange 70 the diameter of which is at least the size of the small diameter of the largest piece 70. With this arrangement the pieces cannot inadvertently fall apart. A mouthpiece 80 is provided with a flared end sized to fit inside piece 78, and with an opening of sufficient diameter to allow a patient to inhale therethrough in all unrestricted manner.

While the invention has been described in connection with what is presently considered to be the most practical and preferred embodiment, it is to be understood that the invention is not limited to the disclosed embodiment, but, on the contrary, is intended to cover various modifications and equivalent included within scope of the appended claims.

## Claims

1. An apparatus for delivering medication to a patient, comprising:
a mouthpiece (10) the proximal end (12) of which is shaped to be accommodated by a human mouth; and a rigid housing (50), having a first end (52), a second end (54), and a main body portion (58) defining a chamber therewithin, said first end (52) having an aperture (56) defined therethrough and in communication with an interior of said mouthpiece (10) so that said chamber is fluidly coupled to said mouthpiece (10) through said aperture (56) in said first end (52)
and the rigid housing (50) having a second aperture (62) through which medication can be introduced into the chamber of the housing (50),
characterised in that:
said rigid housing (50) further has at least one orifice (60 a-i) defined through at least one of said first end (52), said second end (54), and said main body portion (58) for limiting the volumetric flow rate of air into said chamber whereby once medication has been delivered to the interior of the rigid housing (50), a patient breathing through the mouthpiece (10) can inhale medicated air from the chamber and then continue inhaling to his total lung capacity at a limited volumetric flow rate defined by said at least one orifice (60a-i).

2. An apparatus as in claim 1, characterised in that the at least one orifice (60 a-i) limits the volumetric flow of air into said rigid housing (50) to 30 litres per minute or less.

3. An apparatus as in claim 1 or 2, characterised in that there are at least two orifices (60 a-i) and at least one of said orifices (60 a-i) may be selectively closed off for the purpose of limiting the flow of air into said rigid housing (50).

4. An apparatus according to any of claims 1 to 3, further comprising means (57) for closing said second aperture (62) after the aerosolized medication has been introduced into said rigid housing (50).

## Patentansprüche

1. Vorrichtung zum Verabreichen von Medikamenten an einen Patienten, mit:
einem Mundstück (10), dessen proximales Ende (12) so geformt ist, um vom Mund eines Menschen aufgenommen zu werden; und einem festen Gehäuse (50), das ein erstes Ende (52), ein zweites Ende (54) und einen Rumpfhauptabschnitt (58) hat, der eine Kammer darin definiert, wobei das erste Ende (52) eine hindurchgehende und sich in Kommunikation mit dem Inneren des Mundstücks (10) befindliche Öffnung (56) hat, so daß die Kammer durch die Öffnung (56) in dem ersten Ende (52) in Fließverbindung mit dem Mundstück (10) steht, und wobei das feste Gehäuse (50) eine zweite Öffnung (62) hat, durch die Medikamente in die Kammer des Gehäuses (50) eingeführt werden können,
dadurch gekennzeichnet, daß das feste Gehäuse (50) weiter wenigstens einen Durchlaß (60 a-i) hat, der wenigstens durch eines von dem ersten Ende (52), dem zweiten Ende (54) und dem Rumpfhauptabschnitt (58) gebildet ist, um den Volumenstrom von Luft in die Kammer zu begrenzen, wodurch ein durch das Mundstück (10) atmender Patient, sobald Medikament in das Innere des festen Gehäuses (50) eingeführt ist, mit Medikament angereicherte Luft aus der Kammer inhalieren kann und dann das Inhalieren bis zur vollen Lungenkapazität bei einem begrenzten Volumenstrom, der durch den wenigstens einen Durchlaß (60 a-i) definiert ist, fortsetzen kann.

2. Vorrichtung nach Anspruch 1, dadurch gekennzeichnet, daß der wenigstens eine Durchlaß (60 a-i) den Volumenstrom von Luft in das feste Gehäuse (50) auf 30 Liter pro Minute oder weniger begrenzt.

3. Vorrichtung nach Anspruch 1 oder 2, dadurch gekennzeichnet, daß wenigstens zwei Durchlässe (60 a-i) vorhanden sind und daß wenigstens einer der Durchlässe (60 a-i) selektiv verschlossen werden kann, um den Luftstrom in das feste Gehäuse (50) zu begrenzen.

4. Vorrichtung nach einem der Ansprüche 1 bis 3, die weiter eine Einrichtung (57) zum Schließen der zweiten Öffnung (62) nach Einführung von ärosolförmigem Medikament in das feste Gehäuse (50) aufweist.

## Revendications

1. Dispositif pour la délivrance de médicaments à un patient, comprenant :
un embout buccal (10), dont l'extrémité proximale (12) a une forme permettant de la loger dans une bouche humaine, et un boitier rigide (50) ayant une première extrémité (52), une seconde extrémité (54), et une partie de corps principal (58) définissant intérieurement une chambre, ladite première extrémité (52) comportant une ouverture (56) définie dans celle-ci et communiquant avec l'intérieur dudit embout buccal (10), de telle sorte que ladite chambre soit couplée en ce qui concerne les fluides audit embout buccal (10) par l'intermédiaire de ladite ouverture (56) ménagée dans ladite première extrémité (52),
le boîtier rigide (50) ayant une seconde ouverture (62) à travers laquelle le médicament peut être introduit dans la chambre du boîtier (50),
caractérisé en ce que ledit boîtier rigide (50) comporte en outre au moins un orifice (60a à 60i) défini à travers au moins l'une de ladite extrémité (52), ladite seconde extrémité (54) et ladite partie de corps principale (58) pour limiter le débit volumétrique de l'air dans ladite chambre, de sorte que, une fois que le médicament a été délivré à l'intérieur du boîtier rigide (50), un patient respirant à travers l'embout buccal (10) peut inhaler de l'air contenant un médicament à partir de la chambre et ensuite continuer d'inhaler jusqu'à remplir complètement ses poumons à un débit volumétrique limité défini par ledit au moins un orifice (60a à 60i).

2. Dispositif selon la revendication 1, caractérisé en ce que le au moins un orifice (60a à 60i) limite le débit volumétrique de l'air dans ledit boîtier rigide (50) à 30 litres par minute ou moins.

3. Dispositif selon l'une des revendications 1 ou 2, caractérisé en ce qu'il y a au moins deux orifices (60a à 60i) et qu'au moins l'un desdits orifices (60a à 60i) peut être fermé de manière sélective dans le but de limiter l'écoulement d'air dans ledit boîtier rigide (50).

4. Dispositif selon l'une des revendications 1 à 3, comprenant en outre des moyens (57) pour fermer ladite seconde ouverture (62) après que le médicament sous forme d'aérosol a été introduit dans ledit boîtier rigide (50).
